# EUROPEAN PATENT SPECIFICATION

(11) **EP 0 694 306 B1**
(45) Date of publication and mention of the grant of the patent: **03.12.2003**
(21) Application number: 95906498.1
(22) Date of filing: 20.01.1995
(51) Int. Cl.: A61K 35/78, A61P 31/18

(54) **ANTIVIRAL POWDER, ANTIVIRAL EXTRACT, AND PHARMACEUTICAL PREPARATION CONTAINING SAID POWDER AND/OR SAID EXTRACT**
ANTIVIRALES PULVER, ANTIVIRALER EXTRAKT UND PHARMAZEUTISCHE ZUBEREITUNG, DIE BESAGTES PULVER ODER BESAGTEN EXTRAKT ENTHÄLT
POUDRE ANTIVIRALE, EXTRAIT ANTIVIRAL, ET PREPARATION PHARMACEUTIQUE CONTENANT LADITE POUDRE ET/OU LEDIT EXTRAIT

(30) Priority: 20.01.1994 JP 2189594
(43) Date of publication of application: 31.01.1996
(73) Proprietor: Hoashi, Masahito, Nakano-ku, Tokyo 164 (JP)
(72) Inventor: KADONO, Toshiko, Tokyo 132 (JP); SEKINO, Yoshihiro, Kanagawa 251 (JP); OGITA, Zenichi, Toyama 930 (JP)
(74) Representative: Casalonga, Axel
(86) International application number: JP9500059
(87) International publication number: WO95019782

(56) References cited:
- EP-A- 0 568 001
- WO-A-91/06311
- JP-A- 2 172 922
- JP-A- 3 072 490
- JP-A- 4 036 244
- ZHENG MINSHI: "AN EXPERIMENTAL STUDY OF ANTIVIRAL ACTION OF 472 HERBS ON HERPES SIMPLEX VIRUS." JOURNAL OF TRADITIONAL CHINESE MEDICINE, vol. 8, no. 3, September 1988, pages 203-206, XP002059586
- DATABASE WPI Section Ch, Week 8731 Derwent Publications Ltd., London, GB; Class B02, AN 87-216880 XP002059587 & JP 62 142 181 A (NIPPON SHINYAKU CO LTD) , 25 June 1987

## Description

### TECHNICAL FIELD

This invention relates to a process for preparing a leaf portion of Rhus verniciflua or a flower portion of Ulmus davidiana derived antiviral powder, an antiviral extract obtained from said powder, and a preparation comprising said powder and/or extract.

### TECHNICAL BACKGROUND

In the history of human beings who have suffered with various bacterial and viral infections from old, the discovery of antibiotics and the development of vaccines against viruses have brought about a great development in the medical care of this century. However, many chronic and infectious diseases have still presented a serious social problem.

More particularly, the greatest problem which confronts us today includes prophylaxis of infection with viruses such as human immunodeficiency virus (abbreviated as HIV; Barre-Sinoussi et al., 1983; Gallo et al., 1984) which causes human adult T-cell leukemia (abbreviated as HALT) and human T-cell lymphotropic virus (abbreviated as HTLV; Yoshida et al. , 1984) which causes acquired immunological deficiency syndrome (abbreviated as AIDS) and the treatment of these diseases.

Some of herb medicines and crude drugs constituting the herb medicines have been hitherto believed as having the antiviral action. In recent years, a number of studies and reports have been made on crude drugs having the antiviral action. The antiviral activity itself has been elucidated on about half of the crude drugs, thus leaving many crude drugs to be unclear with respect to the activity. The mechanism of the antiviral action has now been under detailed study. Some reports have been already made on the antiviral action through an immunological regulation activity system.

On the other hand, there is the possibility of developing antiviral agents along the line of the central dogma of the molecular biology, i.e. the flow of deoxyribose nuleic acid (abbreviated as DNA)→messenger ribonucleic acid (abbreviated as mRNA)→protein.

More particularly, in the direction opposite to the above-mentioned direction, the transcription which occurs in a reverse direction from the ribonucleic acid (abbreviated as RNA) towards deoxyribonucleic acid takes place in the natural field. This has been backed up with the discovery of a reverse transcriptase (abbreviated as RTV) made by Temin and Baltimore in 1970 (Baltimore, 1970; Temin and Mizutani, 1970).

Retroviruses (reverse transcriptase containing oncovirus) are those having reverse transcriptases as self-explanatory from the name. Specific attention has been made to the virus since the retroviruses have been recognized in 1980's as causing human adult T-cell leukemia and AIDS whose spreading has been viewed with anxiety.

Since then, extensive studies have been made on retroviruses. At present, detailed information has been reported at a gene level and the life cycles of the viruses are now being made clear (Meek et al., 1990a).

The target in the development of antiviral agents is considered to reside in a method of suppressing bond of viruses to cells and reverse transcription activity of from viral ribonucleic acid toward deoxyribonucleic acid. Typical compounds reported as having the activity of inhibiting the reverse transcription include nucleotides, antibiotics, natural matters and the like.

Of these, nucleotide derivatives are greatest in number and have been synthesized for the purpose of the competitive inhibiting action of the reverse transcriptase substrate (Mitsuya and Broder, 1987).

Azidothymidine, 2',3'-dideoxycytidine and 2',3'-dideoxynosin are typical of these reverse transcriptase inhibitors and have been a few medicines permitted as a curing agent for AIDS.

### SUMMARY OF THE INVENTION

An object of the invention is to provide a process for preparing a leaf portion of Rhus verniciflua or a flower portion of Ulmus davidiana derived antiviral powder capable of inhibiting the enzymatic activity of reverse transcriptases and derived from medicinal plants, and a process for preparing on antiviral extract extracting an inhibiting component from said powder.

Said antiviral preparation has an inhibiting action on HIV-I reverse transcriptase which is the pathogen of AIDS and also on a reverse transcriptase derived from mouse leukemia virus.

The antiviral powder derived from a loaf portion of Rhus verniciflua or a flower of Ulmus davidiana is one obtained according to the following procedure. Immediately after collection of the medicinal plant starting material having the antiviral action, the starting material is quickly heated under conditions sufficient to de-activate the enzymatic activity of the starting material, followed by drying the quickly heated plant at a low temperature of from -5°C to +10°C to an extent that a moisture content is not higher than 10% to obtain a powder of the medicinal plant. For obtaining a powder, the medicinal plant is milled or powdered at a stage after the quick heating or after the low temperature drying.

In order to obtain an antiviral extract, the powder of the medicinal plant is subjected to extraction with hot water or a lower alcohol. If necessary, the extract may be appropriately concentrated.

In the practice of the invention, a preparation comprising the powder and/or extract as an effective component is also provided. The preparation may be formulated as an emulsion, oil, wettable powder, powder or the like. Preferably, a powder is used.

### DETAILED DESCRIPTION OF THE INVENTION

The starting medicinal plants used to obtain the antiviral powder or extract and having the antiviral action may be any medicinal plants provided that they have the antiviral activity. The leaves and, preferably, young leaves of Japanese lacquer (Rhus verniciflua) and the flower portion of Ulmus davidiana var. japonica are preferably used as having a remarkable reverse transcriptase inhibiting action. It will be noted that the term "young leaves" used herein means ones which are in a soft condition and which generally are not older than 8 weeks in age, preferably not older than 4 weeks. The above definition concerning the young leaves should not be construed as limitative because the Japanese lacquer greatly differs in the rate of growth depending on the growing district.

In order to obtain the antiviral powder, the certain portions of the medicinal plant are collected. Immediately after the collection, the portions are subjected to quick heating. The term "immediately after collection" is intended to mean that the portions collected are subjected to quick heating within 3 minutes to about 30 minutes after the collection. The immediate quick heating can present an effective component from being decomposed with enzymes to an extent. The terms "quick heating under conditions sufficient to de-activate enzymatic activity" means that when using, for example, a pressure drum heater, the starting material is thermally treated at a temperature ranging from 80 to 140°C within 2 minutes, preferably for approximately one minute, and more preferably for 40 - 50 seconds. Alternatively, a heater such as a microwave oven may also be used. In the case, with an output power of 600 W, the active enzymes can be de-activated by heating for 20 to 50 seconds although depending on the amount of starting material. The quick heating should be carried out under conditions of deactivating a lytic enzyme or enzymes in the starting material but not decomposing other components of the medicinal plant. Since the lytic enzyme is de-activated or broken down, the decomposition of the other components with the lytic enzyme can be prevented, thereby preventing a lowering in amount of effective components.

The thus quickly heated starting material is subsequently subjected to low temperature drying. The low temperature drying is performed within a range of -5 to 10°C. The drying at low temperatures can prevent decomposition in the drying step of the medicinal plant components including an effective component or components. The low temperature drying is continued until the content of moisture in the starting material is not higher than 10 wt%, preferably from 2 to 5 wt%.

For the low temperature drying, one or both of a hot air or air dryer and an ice temperature dryer is used to dry the material within the above-defined temperature range.

The ice temperature dryer is commercially available, for example, from Nippon Light Metal Co., Ltd., as having the following specification.
Temperature range: -5°C ∼ +10°C
Humidity range: 50 ∼ 80% (temperature of 0°C ∼ -5°C)
Air velocity: 1 ∼ 8 m/second

The dimension of the ice temperature drying chamber of the dryer is 96 cm x 53 cm x 59 cm and the capacity of a refrigerator is 2.2 kW with a cooling medium being, for example, Flon Q22.

The hot air dryer includes, for example, a far infrared ray dryer. If this far infrared ray dryer is used, the starting material is subjected to ice temperature drying after completion of the far infrared ray treatment.

The thus dried material is milled by a usual milling device, such as, for example, a colloid mill, to a size of 50 to 100 mesh µm. Alternatively, the materials may be finely cut into powder.

The milling or cutting may not be necessarily performed after completion of the low temperature drying but may be effected after completion of the quick heating. The milling step may be appropriately changed as desired.

The thus obtained antiviral powder may be used as it is or an effective component may be extracted with hot water or a lower alcohol. In the latter case, the low temperature drying is not necessarily required, i.e. the extraction may be effected after the quick heating. For the extraction, an ordinarily employed extractor such as the Soxhlet extractor is used wherein hot water or a lower alcohol is refluxed. Examples of the lower alcohol include aqueous solutions of 30 to 50% of methyl alcohol or ethyl alcohol. The extraction is generally continued for 30 to 60 minutes under reflux. The resultant extract may be concentrated to a level of about 0.1 wt%.

The concentrated extract is a kind of soft extract, is unlikely to handle as it is and should preferably used in the form of a diluted preparation.

More particularly, the powder or extract of the invention may be used in the form of various preparations as having set out hereinbefore and should preferably be used as a powder or wettable powder. Where the antiviral powder or extract is employed in the form of a powder or wettable powder, starch, lactose, dextrin and the like are used as an adjuvant. In this case, the antiviral powder and/or extract is contained in an effective amount. Preferably, the content is not less than 1 wt%, more preferably not less than 10 wt%, of a preparation composition. This content is applicable to other types of preparations.

The inhibiting action of the antiviral powder and/or extract has been investigated as set out in examples appearing hereinafter: different types of medicinal plants have been treated according to the procedure set out hereinbefore and subjected to screening by use of a reverse transcriptase derived from an avian myeloblastosis virus (usually abbreviated as AMV), followed by investigating inhibiting components against a HIV-1 reverse transcriptase which is a pathogen of AIDS and a reverse transcriptase. Moreover, the comparison with the inhibiting action on the reverse transcriptase derived from murine leukemia virus (abbreviated as MULV) was performed. As a result, it has been found that the young leaf portions of the Japanese lacquer which are not older than 8 weeks in age, more preferably 4 ∼ 5 weeks, and the flower portion of Ulmus davidiana var. japonica exhibit a good inhibiting effect.

The invention is more particular described by way of examples and preparation examples.

### Example 1

The effect of plants belonging to the Rhus of Anacardiaceae on inhibition of the reverse transcriptases was checked. Young leaves of Rhus verniciflua, Rhus trichocarpa, Rhus ambigna, Rhus javanica, and Rhus sylverstris were, respectively, collected. Immediately, these leaves were subjected to quick heating for 50 seconds in a microwave oven of 600 W. Thereafter, the leaves were dried at a temperature of 10°C and milled to obtain powders of the respective starting materials. 10 mg of the respective powders obtained in this manner was extracted with 10 ml of hot water for 1 hour. Each hot water extract was checked with respect to the reverse transcriptase inhibiting action according to the following procedure. More particularly, each extract was centrifugally separated to obtain a supernatant liquid, adjusting the sample in such a way that a final concentration in a reaction system was at 50.5 µg/ml, and then subjecting to an RT (reverse transcriptase) inhibition assay. The results are shown in Table 1. This table shows the results of the RT inhibition action of the leaf portions of the respective samples wherein a smaller value exhibits a better inhibiting action.

**Table 1**

| Effect of Plants Belonging to Rhus of Anacardiaceae on Inhibition of Reverse Transcriptase (hot water extract) | |
|---|---|
| hot water extract | extract with ethyl alcohol |
| Rhus verniciflua | 4 ∼ 5 |
| Rhus trichocarpa | 50 |
| Rhus ambigna | 40 |
| Rhus javanica | 20 ∼ 40 |
| Rhus sylvestris | 20 ∼ 40 |

The above results reveal that the hot water extract of the leaves of the Rhus verniciflua exhibit a good reverse transcriptase inhibiting effect. However, the hot water extracts of the other plants belonging to the Rhus of Anacardiaceae have no significant reverse transcriptase effect.

### Example 2

Young and old leaves of the Rhus verniciflua, and young and old leaves and a flower portion of Ulmus davidiana var. japonica were, respectively, used and treated in the same manner as in Example 1 provided that the extraction was effected using both hot water and an aqueous solution of 30% ethyl alcohol. The results are shown in Table 2.

**Table 2**

| Effect of Rhus verniciflua and Ulmus davidiana var. japonica on Inhibition of Reverse Transcriptase (hot water extract) | | |
|---|---|---|
| | hot water extract | extract with ethyl alcohol |
| young leaves of Rhus verniciflua (note 1) | ≦4 ∼ 5 | ≦4 ∼ 5 |
| old leaves of Rhus verniciflua | 100 | 100 |
| young leaves of Ulmus davidiana var. japonica | 50 | 50 |
| old leaves of Ulmus davidiana var. japonica | 100 | 100 |
| flower of Ulmus davidiana var. japonica | 20 | 20 |
| (Note 1) The young leaves of Rhus verniciflua were collected at about the middle of March at Wajima city of Ishikawa Prefecture with their length being 5∼6 cm. | | |

The above results reveal that the reverse transcriptase inhibiting effect is recognized in the hot water extract and the lower alcohol extract (30% ethyl alcohol) of the powders obtained after the quick heating of the young leaves of Rhus verniciflua, but is not recognized for the respective extracts of the old leaves of Rhus verniciflua.

On the other hand, the flower portion of Ulmus davidiana var. japonica has a reverse transcriptase inhibiting effect although not so great.

### Example 3

In this example, a mixture of the young leaves of Rhus verniciflua and the flower portion of Ulmus davidiana var. japonica was checked with respect to the reverse transcriptase inhibiting effect.

More particularly, collected young leaves of Rhus verniciflua and flower portion of Ulmus davidiana var. japonica were immediately quickly heated in a microwave oven of 600 W. The young leaves and the flower portion were mixed at ratios of 1:1 (sample 1), 2:1 (sample 2) and 1:2 (sample 3). Each sample was dried at a low temperature in a drying device, followed by milling. The extracts of the samples with hot water and 30% ethyl alcohol were subjected to measurement of the reverse transcriptase inhibiting action. The results are shown in Table 3.

**Table 3**

| Effect of Extracts of Mixtures of Young Leaves of Rhus verniciflua and Flower Portion of Ulmus davidiana var. japonica on Inhibition of Reverse Transcriptase (IC 50/µg/mg) | | |
|---|---|---|
| | hot water extract | extract with ethyl alcohol |
| sample 1 | 5 | 5 |
| sample 2 | 2∼3 | 2∼3 |
| sample 3 | 3∼4 | 3∼4 |

From the above results, it will be seen that the extracts of the mixtures of the young leaves of Rhus verniciflua and the flower portion of Ulmus davidiana var. japonica improve the inhibition effect presumably by the synergistic effect of the mixture. Accordingly, the mixed extract is a preferred one of the invention.

### Preparation Example 1

Dry powder of 10% young leaves of Rhus verniciflua (1:10 dilution)

| | |
|---|---|
| Dry powder of young leaves of Rhus verniciflua | 100 g |
| 0.1% Blue No. 1 aluminium lake lactose | 0.1 g |
| Lactose | balance |
| Total amount | 1000 g |

### Preparation Example 2

Powder of 10% of a mixture of young leaves of Rhus verniciflua and flower portion of Ulmus davidiana var. japonica (1:10 dilution)

| | |
|---|---|
| Extract of young leaves of Rhus verniciflua | 100 g |
| Extract of flower portion of Ulmus davidiana var. japonica | 50 g |
| Starch | balance |
| Total amount | 1000 g |

The 1:10 dilution of the antiviral powder of the invention or a 1:100 dilution of an antiviral extract obtained from a viscous extract prepared by thermally extracting the antiviral powder with 30% ethyl alcohol and concentrating the extract did not lower in the reverse transcriptase inhibiting action, thus the effect thereof being recognized.

## Claims

1. Process for preparing a leaf portion of *Rhus verniciflua* or a flower portion of *Ulmus davidiana* derived antiviral powder by collecting a leaf portion of *Rhus verniciflua* or a flower portion of *Ulmus davidiana* starting material having an antiviral action, immediately subjecting the collected starting material to quick heating under conditions sufficient to de-activate enzymatic activity in the starting material, drying the thus quickly heated starting material at a low temperature of -5°C to +10°C to an extent that a moisture content is no higher than 10%, and milling the starting material after the quick heating or the drying at the low temperature.

2. Process as defined in Claim 1, wherein said starting material is subjected to quick heating within 30 minutes after the collection of said starting material.

3. Process as defined in Claim 1 or 2 wherein said starting material is quickly heated within 2 minutes thereby deactivating enzymatic activity thereof.

4. A medicinal plant-derived antiviral powder obtainable by the process as defined in Claim 1, wherein said starting material consists of a leaf portion of Rhus verniciflua.

5. A medicinal plant-derived antiviral powder obtainable by the process as defined in Claim 1, wherein said starting material consists of a flower portion of Ulmus davidiana var. japonica.

6. An antiviral preparation comprising not less than 1 wt % of the powder as defined in claim 4 or 5.

7. Process for preparing a leaf portion of *Rhus verniciflua* or a flower portion of *Ulmus davidiana* derived antiviral extract by collecting a leaf portion of *Rhus verniciflua* or a flower portion of *Ulmus davidiana* starting material having an antiviral action, immediately subjecting the collected starting material to quick heating under conditions sufficient to de-activate enzymatic activity in the starting material, drying the thus quickly heated starting material at a low temperature of -5°C to +10°C to an extent that a moisture content is not higher than 10%, milling the starting material after the quick heating or the drying at the low temperature, and extracting the resultant antiviral powder with hot water or a lower alcohol.

8. Process as defined in Claim 7, comprising a further concentration after the extraction.

9. The antiviral extract obtainable by the process as defined in Claim 8, wherein said starting material consists of young leaves of Rhus verniciflua, a flower portion of Ulmus davidiana var.japonica or a mixture thereof.

10. An antiviral preparation comprising not less than 1 wt% of the extract as defined in Claim 9.

11. An antiviral preparation which comprises an extract of a leaf of Rhus verniciflua, a flower of Ulmus davidiana var.japonica or both.

12. An antiviral preparation according to Claim 11 which comprises a leaf of Rhus verniciflua not older than eight weeks in age.

13. An antiviral preparation according to Claim 11 which comprises both an extract of a leaf of Rhus verniciflua and an extract of a flower of Ulmus davidiana var.Japonica.

14. An antiviral preparation according to Claim 13 wherein the weight ratio of an extract of a leaf of Rhus verniciflua and an extract of a flower of Ulmus davidiana var.japonica is about 2 :1.

## Patentansprüche

1. Verfahren zur Herstellung eines antiviralen Pulvers, das aus einem Blattabschnitt von Rhus verniciflua oder einem Blütenabschnitt von Ulmus davidiana abstammt, durch Sammeln eines Ausgangsmaterials eines Blattabschnittes von Rhus verniciflua oder einem Blütenabschnitt von Ulmus davidiana, das antivirale Wirkung hat, sofortiges Unterziehen des gesammelten Ausgangsmaterials raschem Erhitzen unter ausreichenden Bedingungen zum Deaktivieren der enzymatischen Aktivität in dem Ausgangsmaterial, Trocknen des so rasch erhitzten Ausgangsmaterials bei einer niedrigen Temperatur von -5°C bis +10°C bis zu einem Ausmaß, daß ein Feuchtigkeitsgehalt nicht höher 10% vorliegt, und Mahlen des Ausgangsmaterials nach dem raschen Erhitzen oder dem Trocknen bei der niedrigen Temperatur.

2. Verfahren nach Anspruch 1, bei welchem dieses Ausgangsmaterial raschem Erhitzen innerhalb von 30 Minuten nach dem Sammeln dieses Ausgangsmatrerials unterzogen wird.

3. Verfahren nach Anspruch 1 oder 2, bei welchem dieses Ausgangsmaterial innerhalb 2 Minuten rasch erhitzt wird, um hierdurch die enzymatische Aktivität hiervon zu deaktivieren.

4. Medizinisches, von Pflanzen abstammendes, antivirales Pulver, erhältlich durch das in Anspruch 1 definierte Verfahren, bei welchem dieses Ausgangsmaterial aus einem Blattabschnitt von Rhus verniciflua besteht.

5. Medizinisches, von Pflanzen abstammendes, antivirales Pulver, erhältlich durch das in Anspruch 1 definierte Verfahren, bei welchem dieses Ausgangsmaterial aus einem Blütenabschnitt von Ulmus davidiana var. japonica besteht.

6. Antivirale Präparation, umfassend nicht weniger als 1 Gew.-% des Pulvers wie in Anspruch 4 oder 5-definiert.

7. Verfahren zur Herstellung eines antiviralen Extraktes, der aus einem Blattabschnitt von Rhus verniciflua oder einem Blütenabschnitt von Ulmus davidiana abstammt, durch Sammeln eines Ausgangsmaterials eines Blattabschnittes von Rhus verniciflua oder einem Blütenabschnitt von Ulmus davidiana, der antivirale Wirkung hat, sofortiges Unterziehen des gesammelten Ausgangsmaterials raschem Erhitzen unter ausreichenden Bedingungen zum Deaktivieren der enzymatischen Aktivität in dem Ausgangsmaterial, Trocknen des so rasch erhitzten Ausgangsmaterials bei einer niedrigen Temperatur von -5°C bis +10°C bis zu einem Ausmaß, daß ein Feuchtigkeitsgehalt nicht höher 10% vorliegt, Mahlen des Ausgangsmaterials nach dem raschen Erhitzen oder dem Trocknen bei der niedrigen Temperatur und Extrahieren des resultierenden antiviralen Pulvers mit heißem Wasser oder einem niederen Alkohol.

8. Verfahren nach Anspruch 7, umfassend eine weitere Konzentration nach dem Extrahieren.

9. Antiviraler Extrakt, erhältlich nach dem Verfahren wie in Anspruch 8 definiert, bei welchem das Ausgangsmaterial aus jungen Blättern von Rhus verniciflua, einem Blütenabschnitt von Ulmus davidiana var. japonica oder einer Mischung hiervon besteht.

10. Antivirale Präparation, enthaltend nicht weniger als 1 Gew.-% des Extraktes wie in Anspruch 9 definiert.

11. Antivirale Präparation, welche einen Extrakt eines Blattes von Rhus verniciflua, einen Blütenabschnitt von Ulmus davidiana var. japonica oder beide enthält.

12. Antivirale Präparation nach Anspruch 11, welche ein Blatt von Rhus verniciflua mit einem Alter von nicht älter als 10 Tagen umfaßt.

13. Antivirale Präparation nach Anspruch 11, welche beides, einen Extrakt eines Blattes von Rhus verniciflua und einen Extrakt einer Blüte von Ulmus davidiana var. japonica, umfaßt.

14. Antivirale Präparation nach Anspruch 13, bei welcher das Gewichtsverhältnis eines Extraktes eines Blattes von Rhus verniciflua und einem Extrakt einer Blüte von Ulmus davidiana var. japon-ica etwa 2 : 1 ist.

## Revendications

1. Procédé de préparation d'une poudre antivirale dérivée d'une portion de feuille de *Rhus verniciflua* ou d'une portion de fleur de *Ulmus davidiana* consistant à collecter un matériel de départ issu d'une portion de feuille de *Rhus verniciflua* ou d'une portion de fleur de *Ulmus davidiana* ayant une activité antivirale, puis à soumettre immédiatement le matériel de départ collecté à un chauffage rapide dans des conditions suffisantes pour inactiver l'activité enzymatique dans le matériel de départ, puis à sécher ce matériel de départ préalablement chauffé rapidement à une température basse de -5 ° C à +10 ° C jusqu'à ce que la composition en humidité ne dépasse pas 10 %, puis à broyer le matériel de départ après le chauffage rapide ou le séchage à basse température.

2. Procédé selon la revendication 1, dans lequel ledit matériel de départ est soumis à un chauffage rapide dans les 30 minutes après la collecte dudit matériel de départ.

3. Procédé selon la revendication 1 ou 2, dans lequel ledit matériel de départ est chauffé rapidement dans les 2 minutes suivant l'inactivation de l'activité enzymatique.

4. Poudre antivirale dérivée de plante susceptible d'être obtenue par le procédé tel que défini dans la revendication 1, dans lequel ledit matériel de départ consiste en une portion de feuille de *Rhus verniciflua*.

5. Poudre antivirale dérivée de plante susceptible d'être obtenue par le procédé selon la revendication 1, dans lequel ledit matériel de départ consiste en une portion de fleur de *Ulmus davidiana* variété *japonica*.

6. Préparation antivirale comprenant au moins 1 % en poids de poudre tel que défini dans la revendication 4 ou 5.

7. Procédé de préparation d'un extrait antiviral dérivé d'une portion de feuille de *Rhus verniciflua* ou d'une portion de fleur de *Ulmus davidiana* consistant à collecter un matériel de départ d'une portion de feuille de *Rhus verniciflua* ou d'une portion de fleur de *Ulmus davidiana* ayant une action antivirale, à soumettre immédiatement ce matériel de départ collecté à un chauffage rapide dans des conditions suffisantes pour inactiver l'activité enzymatique dans ce matériel de départ, puis à sécher ce matériel de départ rapidement chauffé à une température basse de -5° C à +10 °C jusqu'à ce que la quantité d'humidité ne dépasse pas 10 %, puis à broyer ledit matériel de départ après le chauffage rapide ou le séchage à basse température et à extraire la poudre antivirale résultante avec de l'eau chaude ou un alcool inférieur.

8. Procédé selon la revendication 7, comprenant de plus une concentration après l'extraction.

9. Extrait antiviral susceptible d'être obtenu par le procédé tel que défini dans la revendication 8, dans lequel ledit matériel de départ consiste en de jeunes feuilles de *Rhus verniciflua*, une portion de fleur de *Ulmus davidiana* variété *japonica* ou un mélange des deux.

10. Préparation antivirale comprenant au moins 1 % en poids de l'extrait tel que défini dans la revendication 9.

11. Préparation antivirale comprenant un extrait d'une feuille de *Rhus verniciflua* ou d'une fleur de *Ulmus davidiana* variété *japonica* ou les deux.

12. Préparation antivirale selon la revendication 11, qui comprend une feuille de *Rhus verniciflua* vieille au maximum de 8 semaines.

13. Préparation antivirale selon la revendication 11, comprenant à la fois un extrait de feuille de *Rhus verniciflua* et un extrait de fleur de *Ulmus davidiana* variété *japonica*.

14. Préparation antivirale selon la revendication 13, dans laquelle le rapport en poids de l'extrait de feuille de *Rhus verniciflua* et de l'extrait de fleur de *Ulmus davidiana* variété *japonica* est à peu près 2 :1.
